(19) Europäisches Patentamt
European Patent Office
Office européen des brevets

(11) **EP 3 017 766 B1**

(12) **EUROPEAN PATENT SPECIFICATION**

(45) Date of publication and mention
of the grant of the patent:
**25.05.2022 Bulletin 2022/21**

(21) Application number: **15157910.9**

(22) Date of filing: **06.03.2015**

(51) International Patent Classification (IPC):
**A61B 8/08** (2006.01)

(52) Cooperative Patent Classification (CPC):
**A61B 8/5261; A61B 5/055; A61B 6/5247;
A61B 8/4254; A61B 8/469; A61B 8/485;
A61B 8/5246; G01S 7/52022; G01S 7/52042;
G01S 7/52071; G01S 15/8915; G01S 15/8936;
G01S 15/899; G01S 15/8993;** A61B 8/4405;
(Cont.)

(54) **ULTRASOUND IMAGING APPARATUS AND CONTROL METHOD THEREOF**

ULTRASCHALLABBILDUNGSVORRICHTUNG UND STEUERUNGSVERFAHREN DAFÜR

APPAREIL D'IMAGERIE ULTRASONIQUE ET SON PROCÉDÉ DE CONTRÔLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.11.2014 KR 20140154242**

(43) Date of publication of application:
**11.05.2016 Bulletin 2016/19**

(73) Proprietor: **Samsung Medison Co., Ltd.
Gangwon-do 250-870 (KR)**

(72) Inventors:
• **Shin, Dong Kuk
Gyeonggi-do (KR)**
• **Lee, Hyoung Ki
Gyeonggi-do (KR)**
• **Lim, Hyo Keun
Seoul (KR)**
• **Jeong, Woo Kyoung
Seoul (KR)**

(74) Representative: **Frey, Sven Holger
Lorenz & Kollegen
Patentanwälte Partnerschaftsgesellschaft mbB
Alte Ulmer Strasse 2
89522 Heidenheim (DE)**

(56) References cited:
WO-A1-2013/153968    WO-A1-2014/046263
WO-A1-2014/162966    CN-A- 104 055 536
JP-A- 2013 135 738    US-A1- 2011 066 030
US-A1- 2012 133 663    US-A1- 2012 172 710
US-A1- 2013 116 542

EP 3 017 766 B1

(52) Cooperative Patent Classification (CPC): (Cont.)
G01R 33/4814

2

**Description**

BACKGROUND

1. Field

Embodiments of the present disclosure relate to an ultrasound imaging apparatus of creating ultrasound images, and a control method thereof.

2. Description of the Related Art

An ultrasound imaging apparatus acquires images about the inside of an object by irradiating ultrasonic waves generated from transducers of an ultrasound probe to the object and receiving ultrasonic waves reflected from the object. The ultrasound imaging apparatus has an advantage of high safety compared to X-ray diagnosis apparatuses, since it does not expose patients to radiation, and an advantage that it can display images in real time. For the advantages, the ultrasonic imaging apparatus is widely used.

Meanwhile, a high-resolution medical imaging apparatus means a medical imaging apparatus that can acquire clear images of an object using X-rays, Radio Frequency (RF), etc. except for ultrasonic waves.

As an example of the high-resolution medical imaging apparatus, a Magnetic Resonance Imaging (MRI) apparatus acquires high-resolution medical images for sections of an object by representing the intensities of magnetic resonance signals in response to RF signals generated from a specific intensity of a magnetic field, with contrast.

As another example of the high-resolution medical imaging apparatus, a Computerized Tomography (CT) apparatus acquires section images of a human body by irradiating X-rays to the human body at various angles and reconfiguring X-rays transmitted through the human body through a computer.

If an ultrasound image acquired by the ultrasound imaging apparatus and a high-resolution medical image acquired by the high-resolution medical imaging apparatus are converted (hereinafter, referred to as image matching) and displayed on the same coordinate system, a user can easily understand the corresponding relation of the different images.

US 2012/133663 A1 relates to a medical image display apparatus for displaying medical images of a biological tissue and a method for displaying medical images.

US 2013/116542 A1 concerns methods and devices for medical imaging.

WO 2014/046263 A1 provides an image processing device, an X-ray diagnostic device and a display method.

JP 2013 135738 A relates to a surgery support system that continuously monitors the operator's fatigue, surgery instrument guidance position accuracy, and treatment area by recording and analyzing surgery information and contributes to improvement of surgery accuracy.

US 2012/172710 A1 relates to quantitative elastography.

CN 104 055 536 A concerns a medical image processing, in particular a fusion and magnetic resonance image fusion registration method.

WO 2013/153968A1 provides an ultrasonic diagnosis device.

US 2011/066030 A1 relates to medical imaging and in particular to imaging tissue based on shear acoustic waves.

WO 2014/162966 A1 relates to an ultrasonic diagnostic apparatus and to a technique for evaluating elasticity of a living body using acoustic radiation force.

SUMMARY

Therefore, it is an aspect of the present disclosure to provide an ultrasound imaging apparatus of matching an ultrasound image of an object with a high-resolution medical image of the object, and detecting a degree of elasticity of a point of interest using ultrasonic waves to display an ultrasound elastic image together with the ultrasound image and the high-resolution medical image.

The invention in defined by claim 1. Regarding the control method the invention is defined by claim 12.

In accordance with one aspect of the present disclosure, an ultrasound imaging apparatus includes: an image creator configured to create an ultrasound elastic image that represents a degree of elasticity for a region of interest including a predetermined point of interest of an object; and a display configured to display the ultrasound elastic image together with an ultrasound image for the object and a high-resolution medical image matching with the ultrasound image.

The ultrasound imaging apparatus further includes: a connector connected to an ultrasound probe; and a controller configured to determine an irradiation focal point of the ultrasound probe in order to acquire the ultrasound elastic image.

The controller may decide an offset between the irradiation focal point and the point of interest in order to determine the irradiation focal point.

The controller may decide the offset between the irradiation focal point and the point of interest, based on an amount of change in axis direction of shear waves irradiated from the irradiation focal point.

The controller may determine, as the irradiation focal point, a point located at the same depth as the point of interest and spaced by the offset in a lateral direction from the point of interest.

The controller may determine an irradiation width of the ultrasound probe according to a predetermined number of irradiation focal points.

The ultrasound imaging apparatus further includes an ultrasound probe configured to irradiate a focus beam to the irradiation focal point so as to transfer shear waves from the irradiation focal point to the point of interest.

The high-resolution medical image may be at least one image of a magnetic resonance image and an X-ray image.

The ultrasound imaging apparatus further includes a connector connected to an ultrasound probe; and a controller configured to match the ultrasound image with the high-resolution medical image. The ultrasound imaging apparatus may further include a storage in which at least one high-resolution medical image is stored.

The ultrasound imaging apparatus may further include an input configured to receive a user's input of inputting at least one of coordinate information and a feature point of the ultrasound image and at least one of coordinate information and a feature point of the high-resolution medical image, wherein the controller matches the ultrasound image with the high-resolution medical image, based on the user's input.

The connector receives an ultrasound signal and a location signal of the ultrasound probe, from the ultrasound probe, the image creator creates an ultrasound image based on the ultrasound signal, and the controller detects a high-resolution medical image corresponding to the ultrasound image in real time, based on the location signal of the ultrasound probe.

The display may display a predetermined color according to a degree of elasticity of the predetermined point of interest, as the ultrasound elastic image.

The display may display a spectral image based on a degree of elasticity of the predetermined point of interest, as the ultrasound elastic image.

The ultrasound imaging apparatus may further include an input configured to receive a user's input of setting a point in the ultrasound image or the high-resolution medical image to a point of interest, wherein the image creator creates an ultrasound elastic image for the point of interest set according to the user's input.

In accordance with one aspect of the present disclosure, a control method of an ultrasound imaging apparatus includes: creating an ultrasound image for an object; displaying the ultrasound image together with a high-resolution medical image stored in advance; creating an ultrasound elastic image that represents a degree of elasticity for a region of interest including a predetermined point of interest of the object; and displaying the ultrasound elastic image together with the ultrasound image and a high-resolution medical image matching with the ultrasound image.

The creating of the ultrasound elastic image includes determining an irradiation focal point of a ultrasound probe connected to the ultrasound imaging apparatus.

The determining of the irradiation focal point of the ultrasound probe may include deciding an offset between the irradiation focal point and the point of interest.

The determining of the irradiation focal point of the ultrasound probe may include determining an irradiation width of the ultrasound probe according to a predetermined number of irradiation focal points.

The creating of the ultrasound elastic image includes irradiating a focus beam to an irradiation focal point of an ultrasound probe connected to the ultrasound imaging apparatus so as to transfer shear waves from the irradiation focal point to the point of interest.

The control method further includes, before displaying the ultrasound elastic image, matching the ultrasound image with the high-resolution medical image.

BRIEF DESCRIPTION OF THE DRAWINGS

These and/or other aspects of the disclosure will become apparent and more readily appreciated from the following description of the embodiments, taken in conjunction with the accompanying drawings of which:

FIG. 1 is a perspective view of an ultrasound imaging apparatus according to an embodiment of the present disclosure;
FIG. 2 is a control block diagram of an ultrasound imaging apparatus according to an embodiment of the present disclosure;
FIG. 3 is a view for describing a coordinate system of ultrasonic waves that are irradiated from a transducer module and a coordinate system of an object;
FIG. 4 is a plane view showing an ultrasound probe according to an embodiment of the present disclosure on the xz plane in a probe-based coordinate system and an object on the yz plane in a Digital Imaging and Communication in Medicine (DICOM) coordinate system;

FIG. 5 is a view for describing a process of generating shear waves in an object;

FIG. 6 shows the yz planes of an object in the DICOM coordinate system for describing traveling of shear waves;

FIG. 7 is a view for describing traveling of shear waves;

FIGS. 8 and 9 show ultrasound images, ultrasound elastic images, and high-resolution medical images that are displayed through a display;

FIG. 10 shows high-resolution medical images combined into a 3Dimensional (3D) volume image;

FIG. 11 is a view for describing a method of matching a high-resolution medical image with a reference ultrasound image, according to an embodiment of the present disclosure;

FIG. 12 is a view for describing a method of estimating coordinate information of a reference ultrasound image in the DICOM coordinate system, according to an embodiment of the present disclosure;

FIG. 13 is a view for describing a method of guiding movements of an ultrasound probe, according to an embodiment of the present disclosure;

FIG. 14 shows an yz plane of an object for describing an offset between an irradiation focal point and a point of interest;

FIG. 15 shows an xz plane of an object for describing an offset between an irradiation focal point and a point of interest;

FIG. 16 is a view for describing the number of irradiation focal points of a focus beam according to irradiation widths of ultrasonic waves;

FIG. 17 is a flowchart illustrating a control method of an ultrasound imaging apparatus, according to an embodiment of the present disclosure; and

FIG. 18 is a flowchart illustrating a method of creating an ultrasound elastic image in an ultrasound imaging apparatus, according to an embodiment of the present disclosure.

## DETAILED DESCRIPTION

Purposes, specified advantages, and new features of the present disclosure will be apparent by referring to the following detailed description and embodiments described below in connection with the accompanying drawings. Also, like reference numerals refer to like elements throughout. In the following description, if it is determined that detailed descriptions for related art make the subject matter of the present disclosure obscure unnecessarily, the detailed descriptions will be omitted. In this specification, it will be understood that, although the terms first, second, etc. may be used herein to describe various components, these components should not be limited by these terms. These terms are only used to distinguish one component from another.

Hereinafter, an ultrasound imaging apparatus and a control method thereof according to embodiments of the present disclosure will be described in detail with reference to the accompanying drawings.

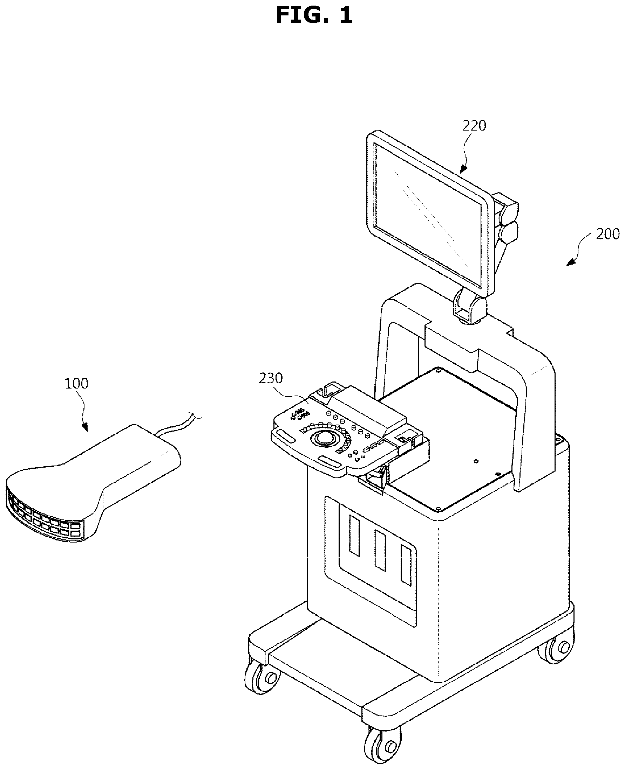

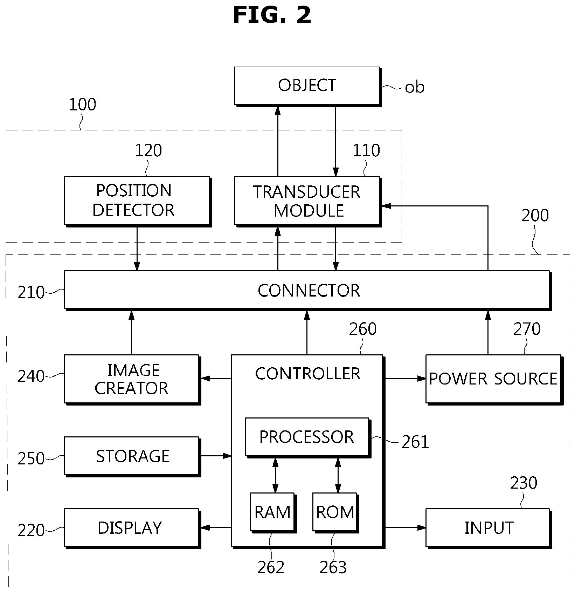

FIG. 1 is a perspective view of an ultrasound imaging apparatus according to an embodiment of the present disclosure, and FIG. 2 is a control block diagram of an ultrasound imaging apparatus according to an embodiment of the present disclosure.

Referring to FIGS. 1 and 2, an ultrasound imaging apparatus includes an ultrasound probe 100 to irradiate ultrasonic waves to an object ob, to receive ultrasonic waves reflected from the object ob, and to convert the received ultrasonic waves into electrical signals (hereinafter, referred to as ultrasonic signals), and may include a main body 200 to create ultrasound images based on the ultrasonic signals. The main body 200 may be a workstation including a connector 210 connected to the ultrasound probe 100, a display 220 to display ultrasound images, and an input 230 to receive a user's manipulations.

Hereinafter, the ultrasound probe 100 will be first described, and the main body 200 will be described later.

The ultrasound probe 100 may collect information about a target region of the object ob using ultrasonic waves.

The ultrasound probe 100 may include a transducer module 110 to generate ultrasonic waves, to irradiate the ultrasonic waves to a target region in the object ob, and to receive ultrasonic waves (hereinafter, referred to as reflected ultrasonic waves) reflected from the target region, and a position detector 120 to detect movements of the ultrasound probe 100.

The transducer module 110 may generate ultrasonic waves according to a received pulse signal or a received alternating current signal, and irradiate the ultrasonic waves to the object ob. The ultrasonic waves irradiated to the object ob may be reflected from a target region in the object ob. The transducer module 110 may receive the ultrasonic waves reflected from the object ob, and convert the received ultrasonic waves into electrical signals to generate ultrasonic signals.

More specifically, the transducer module 110 may receive a voltage from an external power source or an internal power source (e.g., a battery). For example, the transducer module 110 may receive a voltage from a power source 270 in the main body 200. If the transducer module 110 receives a voltage, piezoelectric vibrators or thin films constituting the transducer module 110 may vibrate.

Then, the transducer module 110 may irradiate ultrasonic waves generated by the vibrations of the piezoelectric vibrators or thin films to the object ob.

The transducer module 110 may irradiate ultrasonic waves to a location corresponding to specific coordinate information according to a user's manipulation.

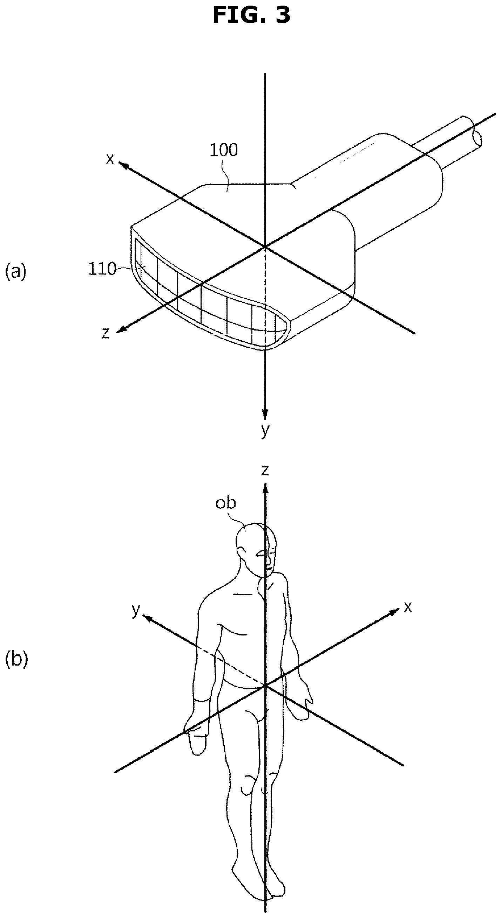

FIG. 3 is a view for describing a coordinate system of ultrasonic waves that are irradiated from the transducer module 110 and a coordinate system of the object ob.

As shown in (a) of FIG. 3, it is assumed a probe-based coordinate system in which a direction from the lower part of the ultrasound probe 100 to the upper part, which is the direction of ultrasonic waves irradiation by the transducer module 110, is a direction in which z-axis values increase, a direction from the front side of the ultrasound probe 100 to the rear side is a direction in which y-axis values increase, and a direction from the left part of the ultrasound probe 100 to the right part is a direction in which x-axis values increase.

The direction in which z-axis values increase may be defined as an axis direction, the direction in which x-axis values increase may be defined as a lateral direction, and the direction in which y-axis values increase may be defined as an elevation direction.

More specifically, a direction in which ultrasound waves are irradiated vertically may be defined as an axis direction, a direction in which a plurality of transducers are aligned in a line may be defined as a lateral direction, and a direction perpendicular to the axis direction and the lateral direction may be defined as an elevation direction.

Coordinate information may be information about at least one of a direction, a tilt angle, and a rotation angle in a space.

Coordinate information of the ultrasound probe 100 may be acquired by the position detector 120 attached on or provided outside the ultrasound probe 100. If coordinate information of the ultrasound probe 100 is acquired, a controller 260 which will be described later may estimate coordinate information of an irradiation focal point of ultrasonic waves that are irradiated from the transducer module 110, based on the coordinate information of the ultrasound probe 100.

The position detector 120 may acquire information about a direction, a tilt angle, and a rotation angle of the ultrasound probe 100.

More specifically, the position detector 120 may determine an azimuth (that is, north, south, east, and west) which corresponds to a direction of ultrasonic waves to be irradiated and which an axis of the ultrasound probe 100 indicates.

Also, the position detector 120 may determine an angle which corresponds to a tilt angle of ultrasonic waves to be irradiated and which an axis of the ultrasound probe 100 forms with respect to the earth's axis.

Also, the position detector 120 may determine an angle which corresponds to a rotation angle of ultrasonic waves to be irradiated and which an axis of the ultrasound probe 100 forms with respect to a horizontal plane.

For example, in the ultrasound probe 100 as shown in (a) of FIG. 3, the position detector 120 may determine an azimuth (that is, north, south, east, and west) which corresponds to a direction of ultrasonic waves to be irradiated and which the z axis of the ultrasound probe 100 indicates.

Also, the position detector 120 may determine an angle which corresponds to a tilt angle of ultrasonic waves to be irradiated and which the y axis of the ultrasound probe 100 forms with respect to the earth's axis.

Also, the position detector 120 may determine an angle which corresponds to a rotation angle of ultrasonic waves to be irradiated and which the x axis of the position detector 100 forms with respect to the horizontal plane.

The position detector 120 may be a magnetic sensor, an optical sensor, a resistive sensor, or a plastic sensor to detect various directions, tilt angles, and rotation angles, although it is not limited to these.

In order to describe a coordinate system of the object ob, a coordinate system (hereinafter, referred to as a Digital Imaging and Communication in Medicine (DICOM) coordinate system) that is defined in the DICOM standard will be described as an example, below.

As shown in (b) of FIG. 3, the DICOM coordinate system may be defined by an x axis extending from the right part of the object ob to the left part, an y axis extending from the anterior part of the object ob to the posterior part, and a z axis extending from the inferior part of the object ob to the superior part.

Accordingly, coordinate information in the DICOM coordinate system is characterized in that x-axis values increase from the right part of the object ob to the left part, y-axis values increase from the anterior part of the object ob to the posterior part, and z-axis values increase from the inferior part of the object ob to the superior part.

As another example, coordinate information can be defined based on standard planes of the object ob.

The standard planes of the object ob may include a coronal plane, a transverse plane, and a sagittal plane.

Coordinate information based on the standard planes is characterized in that the coronal plane of an object is set to the xzplane, the transverse plane of the object is set to the xy plane, and the sagittal plane of the object is set to the yz plane, in the DICOM coordinate system.

In the following description, it is assumed that the transducer module 110 irradiates ultrasonic waves in the direction of the sagittal plane (that is, the yz plane) of an object ob.

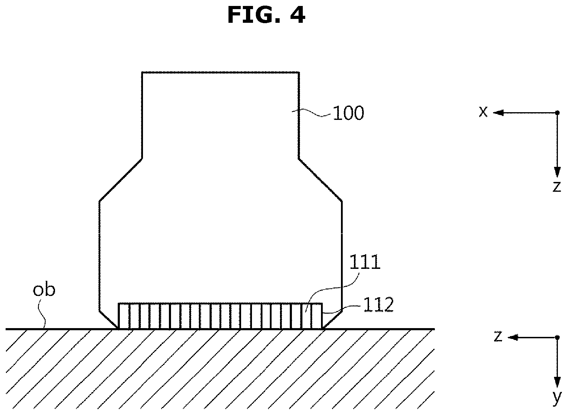

FIG. 4 is a plane view showing the ultrasound probe 100 according to an embodiment of the present disclosure on the xz plane in the probe-based coordinate system and an object on the yz plane in the DICOM coordinate system.

The transducer module 110 (see FIG. 3) of the ultrasound probe 100 may contact the surface of an object ob to irradiate ultrasonic waves to the object ob.

The transducer module 110 may include a transducer array 112 consisting of a plurality of transducers 111, as shown in FIG. 4.

Each transducer 111 may include a piezoelectric element.

The transducers 111 may irradiate ultrasonic waves to the object ob in response to an electrical signal for acquiring an "ultrasound image", and receive reflected ultrasonic waves from the object ob.

The transducer array 112 may be a 1Dimensional (1D) array or a 2Dimensional (2D) array. Hereinafter, the transducer array 112 is assumed to be a 1D array.

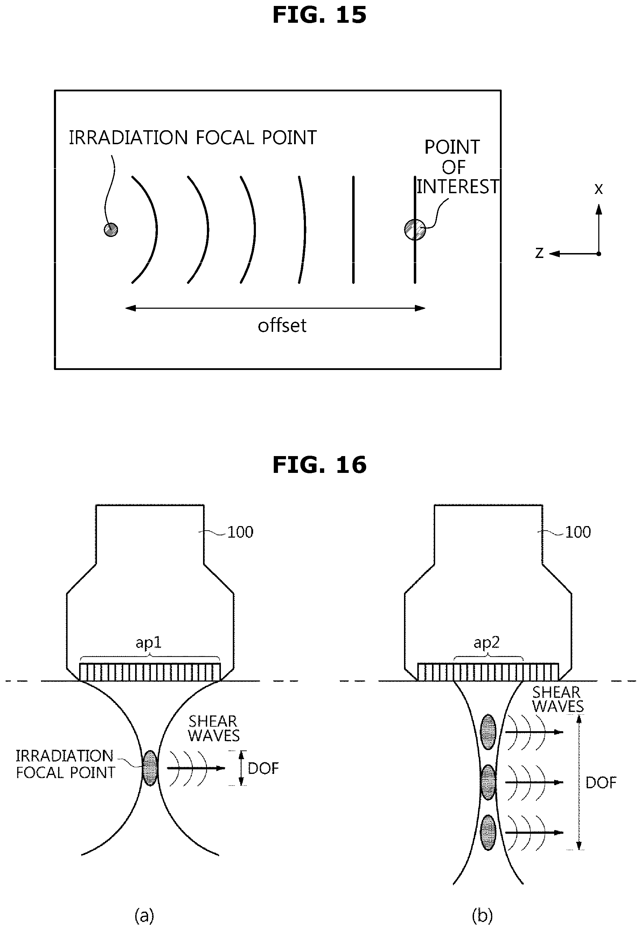

Transducers 111 selected by the controller 260 of the main body 200 from among the plurality of transducers 111 may be activated. The controller 260 may change a range (ap, see FIG. 16) of transducers 111 that are activated so as to change a transmission/reception range of ultrasonic waves according to the range of the activated transducers 111.

The range of transducers 111 that are activated will be referred to as an activation range.

If the transducers 111 receive reflected ultrasonic waves from the object ob, the piezoelectric vibrators or thin films constituting the transducers 111 may vibrate in response to the received ultrasonic waves. Then, the transducers 111 may generate alternating current of a frequency corresponding to the vibration frequency of the piezoelectric vibrators or thin films so as to convert the ultrasonic waves into an electrical signal (that is, an ultrasound signal).

The ultrasound signal is transferred to an image creator 240 (see FIG. 2) through the connector 210 of the main body 200.

Meanwhile, the transducers 111 may irradiate ultrasonic waves to the object ob to generate shear waves that are transferred from an irradiation focal point to the object ob. The ultrasonic waves that are irradiated from the transducers 111 in order to generate shear waves may have a lower frequency than that of ultrasonic waves that are irradiated in order to acquire an ultrasound image.

The ultrasonic waves that are irradiated to the irradiation focal point in order to generate shear waves will be hereinafter referred to as a "focus beam".

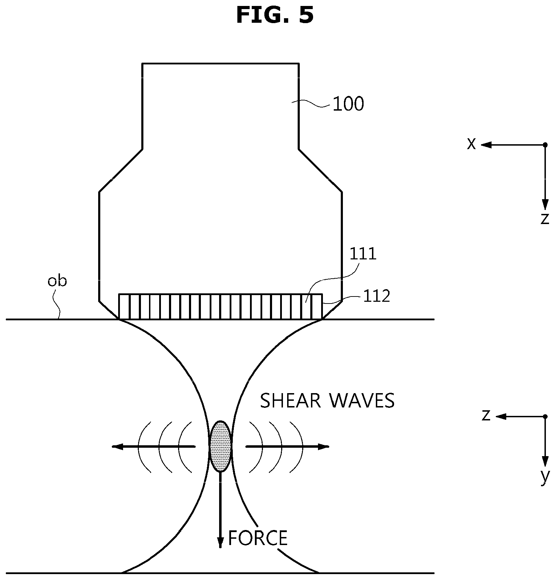

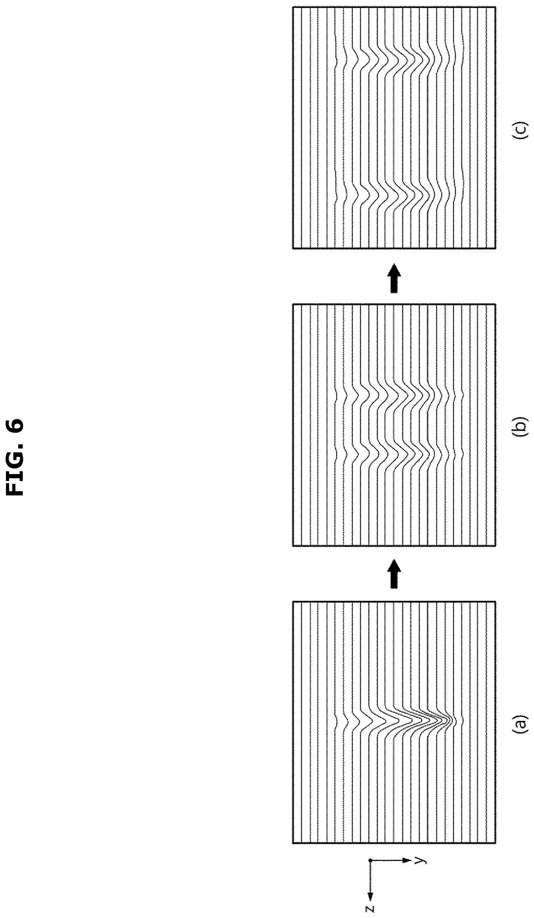

FIG. 5 is a view for describing a process of generating shear waves in an object ob, and FIG. 6 shows the yz planes of an object in the DICOM coordinate system for describing traveling of shear waves.

As shown in FIG. 5, if a focus beam is irradiated to an object ob, a force may be applied in a depth direction to the object ob. If the force is applied in the depth direction to the object ob, as shown in FIG. 6, the tissue of the object ob at a location (that is, an irradiation focal point) to which the force is applied may move in the depth direction (for example, the y-axis direction of the object ob) (see (a) of FIG. 6), and the movement aspect may move in a traverse direction (for example, the +/- z-axis direction of the object ob) perpendicular to the depth direction (see (b) and (c) of FIG. 6). The movement aspect of tissue moving in the traverse direction is called shear waves.

If the controller 260 of the main body 200 measures propagation velocity of the shear waves, a shear modulus of medium through which the shear waves are propagated can be calculated, and the display 220 may quantitatively display stiffness of the tissue based on the shear modulus. An image displaying stiffness of tissue quantitatively is referred to as an ultrasound elastic image.

The transducers 111 may irradiate a focus beam to the irradiation focal point to generate shear waves that are transferred from the irradiation focal point to the object ob, in order to acquire an "ultrasound elastic image". The irradiation focal point may be a part of body tissue, such as a breast, a liver, a kidney, and muscles.

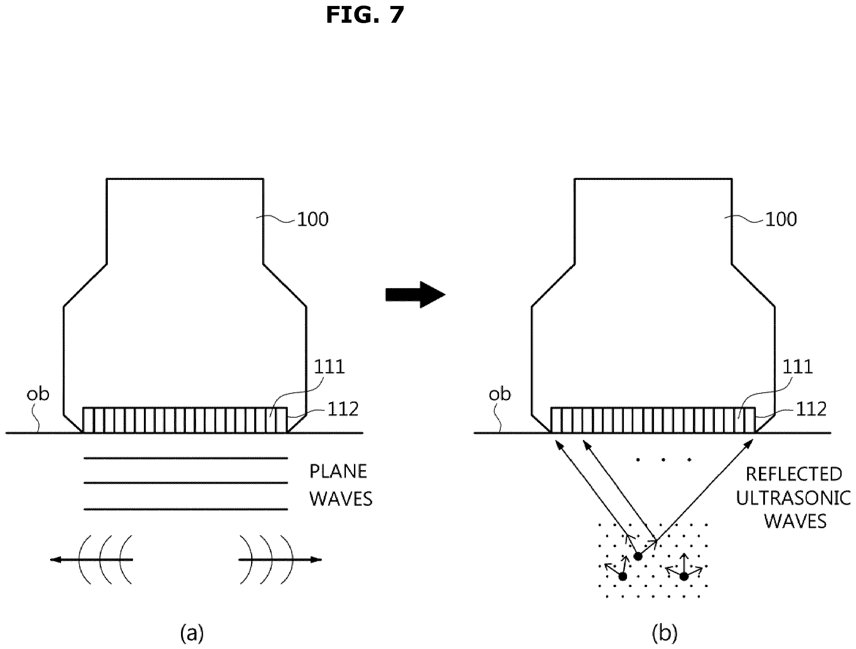

FIG. 7 is a view for describing traveling of shear waves.

If shear waves are generated, the transducers 111 may irradiate ultrasonic waves to an object ob (see (a) of FIG. 7), and receive reflected ultrasonic waves from the object ob (see (b) of FIG. 7) to generate an ultrasound elastic signal that is an electrical signal corresponding to the reflected ultrasonic waves.

If the transducers 111 receive the reflected ultrasonic waves from the object ob, the piezoelectric vibrators or thin films constituting the transducers 111 may vibrate in response to the received ultrasonic waves. Then, the transducers 111 may generate alternating current of a frequency corresponding to the vibration frequency of the piezoelectric vibrators or thin films to convert the ultrasonic waves into an electrical signal (that is, an ultrasound elastic signal).

The ultrasound elastic signal is transferred to the image creator 240 through the connector 210 of the main body 200.

The ultrasound probe 100 may be connected to one end of a cable, and the other end of the cable may be connected to a male connector (not shown).

The male connector connected to the other end of the cable may be physically coupled with a female connector (not shown) of the main body 200.

Hereinafter, components constituting the main body 200 of the ultrasound imaging apparatus will be described.

Referring again to FIGS. 1 and 2, the main body 200 may include the connector 210 connected to the ultrasound probe 100, the display 220 to display ultrasound images, and the input 230 to receive a user's manipulations.

The connector 210 receives an ultrasound signal generated by the ultrasound probe 100, and transfer the ultrasound signal to the image creator 240. For example, the connector 210 receives an ultrasound signal and an ultrasound elastic signal generated by the ultrasound probe 100.

Also, the connector 210 may transfer a control signal generated by the controller 260 to the ultrasound probe 100.

Also, the connector 210 may connect to the power source 270 of the main body 200 to supply a voltage for driving the ultrasound probe 100 to the ultrasound probe 100.

The connector 210 may include one or more female connectors (not shown) that are physically coupled with the male connector (not shown) connected to the cable of the ultrasound probe 100 to transmit/receive signals between the main body 200 and the ultrasound probe 100.

However, the connector 210 may connect the main body 200 to the ultrasound probe 100 through a wired/wireless network.

The wired network may be a Local Area Network (LAN), a Wide Area Network (WAN), or a Value Added Network (VAN), and the wireless network may be a mobile radio communication network, a satellite communication network, Bluetooth, Wireless Fidelity (Wi-Fi), Near Field Communication (NFC), Radio-Frequency Identification (RFID), Infrared Data Association (IrDA), or Zigbee.

The display 220 may display ultrasound images and ultrasound elastic images created by the image creator 240 and high-resolution medical images stored in the storage 250 so that a user can visually examine the internal structure or tissue of an object ob.

The user may be a person who diagnoses an object ob using the ultrasound imaging apparatus, and may be a medical professional, such as a doctor, a radiogical technologist, or a nurse. However, the user is not limited to them, and may be anyone who uses the ultrasound imaging apparatus.

The high-resolution medical images may have been stored in advance in the storage 250, and may be medical images with high resolution and high contrast, such as Magnetic Resonance Imaging (MRI) images, Computed Tomography (CT) images, mammography images, X-ray images, or Positron Emission Tomography (PET) images, except for ultrasound images.

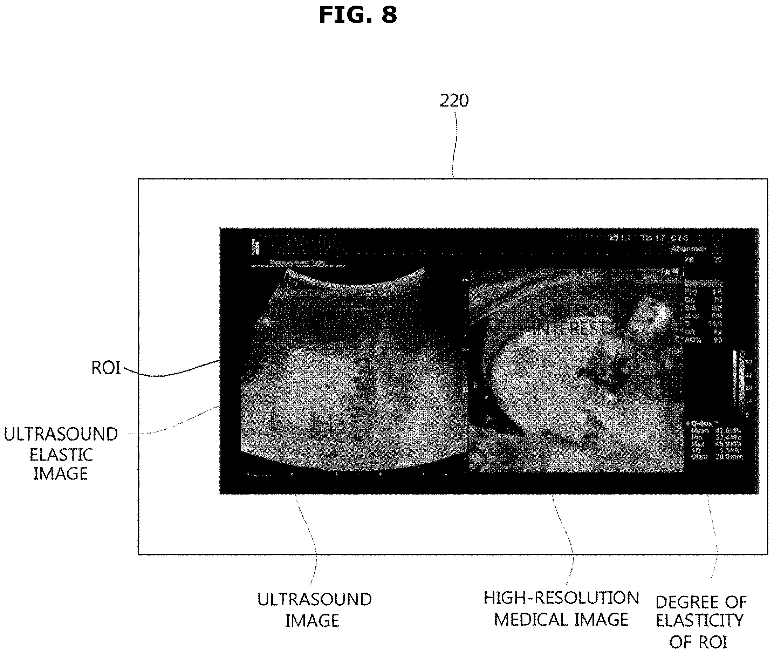

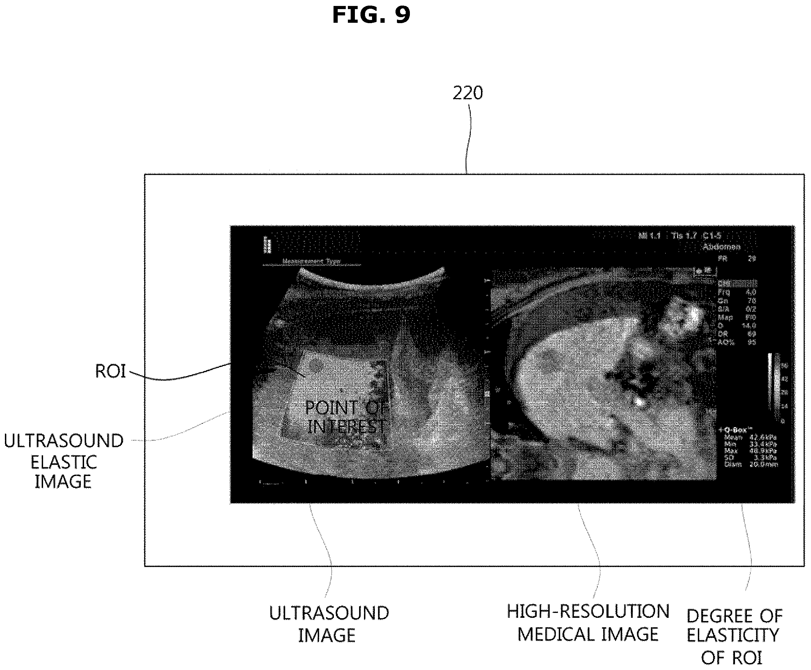

FIGS. 8 and 9 show ultrasound images, ultrasound elastic images, and high-resolution medical images that are displayed through the display 220.

Referring to FIG. 8, the display 220 may display an ultrasound image on the left of a screen, and a high-resolution medical image corresponding to the ultrasound image on the right of the screen. Also, the display 220 may overlap an ultrasound elastic image with the ultrasound image.

In this case, the display 220 may display an ultrasound elastic image for a predetermined Region Of Interest (ROI) set from a predetermined point of interest.

The predetermined point of interest may be coordinate information of a predetermined location of an object ob, stored in the storage 250, or may be coordinate information of a location of an object ob input through the input 230 according to a user's manipulation.

The predetermined ROI may be a region which is within a predetermined distance from the predetermined point of interest.

For example, as shown in FIG. 8, if a user selects a point in a high-resolution medical image, the display 220 may display an ultrasound elastic image for a ROI of an object ob that is that is within a predetermined distance from the selected point.

Also, as shown in FIG. 9, if a user selects a point in an ultrasound image, the display 220 may display an ultrasound elastic image for a ROI of an object ob that is within a predetermined distance from the selected point.

The display 220 may display, as an ultrasound elastic image, predetermined colors according to degrees of elasticity (that is, shear moduli) of the individual points of the ROI.

For example, the display 220 may display a point of a low degree of elasticity such as a tumor with red and a point of a high degree of elasticity with blue, in an ultrasound elastic image. However, the predetermined colors are not limited to the red and blue, and may be set to various colors according to a user's settings.

Also, the display 220 may display numerals representing degrees of elasticity of the individual points of a ROI.

The display 220 may be fixed on the main body 200, or removably coupled with the main body 200.

Although not shown in FIG. 1, a sub display to display applications (for example, menus or guide data needed for ultrasound diagnosis) related to operations of the ultrasound imaging apparatus may be additionally provided.

The display 220 may display various information related to the ultrasound imaging apparatus, and may be implemented as a Liquid Crystal Display (LCD), a Light Emitting Diodes (LED) display, an Organic Light Emitting Diodes (OLED) display, an Active Matrix Organic Light Emitting Diodes (AMOLED) display, a flexible display, or a 3D display. Also, the display 220 may be a touch screen with both a display function and an input function.

The input 230 may receive commands or manipulations related to operations of the ultrasound imaging apparatus, from a user.

For example, the user may input a command for starting ultrasound diagnosis, a command for selecting a point of interest, a command for selecting a mode for ultrasound images to be displayed, or information required for image matching which will be described later, through the input 230.

Modes for ultrasound images may include an Amplitude mode (A-mode), a Brightness mode (B-mode), a Doppler mode (D-mode), an Elastography mode (E-mode), and a Motion mode (M-mode).

The input 230 may include at least one of a keyboard, a mouse, a trackball, a touch screen, a foot switch, and a foot pedal, although it is not limited to these.

The input 230 may be mounted on the main body 200 as shown in FIG. 1, however, the input 230 may be provided below the main body 200 if the input 230 is implemented as a foot switch or a foot pedal.

Also, if the input 230 is implemented as a Graphical User Interface (GUI) such as a touch screen, that is, if the input 230 is implemented with software, the input 230 may be displayed through the display 220.

Referring to FIG. 2, the main body 200 may further include the image creator 240 to create ultrasound images and ultrasound elastic images, the storage 250 to store high-resolution medical images, the controller 260 to generate control signals for controlling components of the main body 200 and the ultrasound probe 100, and the power supply 270 to supply a voltage to the main body 200 and the ultrasound probe 100.

The image creator 240 creates an ultrasound image of an object ob, based on an ultrasound signal received from the ultrasound probe 100 through the connector 210.

Also, the image creator 240 creates an ultrasound elastic image for a ROI of the object ob, based on an ultrasound elastic signal received from the ultrasound probe 100 through the connector 210.

More specifically, the image creator 240 may receive an ultrasound elastic signal from the activated transducers 111 of the ultrasound probe 100 to create an ultrasound image of the object ob.

Since shear waves are propagated at high speed of maximally about 10m per second, the image creator 240 may store variation of tissue in an object ob at high speed of about 5000Hz, using the transducer array 112 of the ultrasound probe 100. The image creator 240 may create a B-mode image.

The image creator 240 may calculate a disparity image from two successive ultrasound images, detect a point at which variation of tissue has occurred, and calculate movement speed of the variation. At this time, the image creator 240 may compare two successive ultrasound images to each other to calculate a degree of variation of scatters, thereby detecting variation of tissue.

Successively, the image creator 240 may determine points of shear waves in each disparity image, and measure propagation velocity of the shear waves.

Then, the image creator 240 may calculate a shear modulus based on the propagation velocity of the shear waves. More specifically, the image creator 240 may calculate a shear modulus by multiplying the square of the propagation velocity of the shear waves by the density of medium.

The image creator 240 may calculate the shear modulus as a degree of elasticity, and create an ultrasound elastic image based on the degree of elasticity.

The ultrasound elastic image may include a predetermined color according to a degree of elasticity of a ROI, as described above, and may be a 3D image or a spectral image that is displayed as a waveform.

The storage 250 may store one or more high-resolution medical images. The high-resolution medical images may be various medical images, such as MRI images, CT images, mammography images, X-ray images, or PET images, except for ultrasound images.

The high-resolution medical images may be combined into 3D volume images and stored.

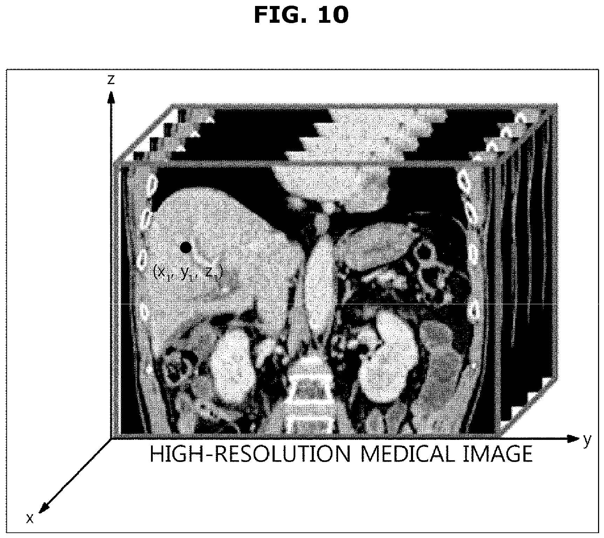

FIG. 10 shows high-resolution medical images combined into a 3D volume image. The storage 250 (see FIG. 2) may store 3D volume images corresponding to various regions of an object. Each 3D volume image may be created by combining one or more section images with each other.

Also, the storage 250 may store image information included in each high-resolution medical image. For example, as shown in FIG. 10, the storage 250 may store information representing that a high-resolution medical image being a section image corresponds to the coronal plane (that is, the yz plane) in the DICOM coordinate system obc, and coordinate information (For example, (x1, y1, z1)) of a specific point included in the high-resolution medical image.

Also, the storage 250 may store ultrasound images and ultrasound elastic images created by the image creator 240, information about predetermined points of interest or points of interest input by a user, and information about predetermined ROIs. Also, the storage 250 may store various information required for controlling the ultrasound imaging apparatus.

The storage 250 may be a cache, Read Only Memory (ROM), Programmable Read Only Memory (PROM), Erasable Programmable Read Only Memory (EPROM), Electrically Erasable Programmable ROM (EEPROM), a non-volatile memory device such as flash memory, a volatile memory device such as Random Access Memory (RAM), a Hard Disk Drive (HDD), or Compact Disk-ROM (CD-ROM), although it is not limited to these.

Referring again to FIG. 2, the controller 260 may generate control signals for controlling the components of the ultrasound imaging apparatus.

Hereinafter, a control method that is performed by the controller 260 will be described.

The controller 260 may control the power source 270 to supply a voltage to the ultrasound probe 100 in order to drive the ultrasound probe 100.

Successively, the controller 260 may determine an activation range of the transducers 111 of the ultrasound probe 100, and control the transducers 111 belonging to the activation range to irradiate ultrasonic waves to an object ob. The activation range may have been set in advance and stored in the storage 250, or may be received from a user through the input 230.

Then, the controller 260 may control the transducers 111 to receive reflected ultrasonic waves from the object ob.

Thereafter, the controller 260 may control the image creator 210 to create an ultrasound image based on the ultrasound signals received by the transducers 111.

The "ultrasound image" may be selected according to a user's input or a predetermined setting, and used as a "reference ultrasound image" matching with a high-resolution medical image.

Then, the controller 260 matches the reference ultrasound image created by the image creator 210 with a high-resolution medical image stored in the storage 250.

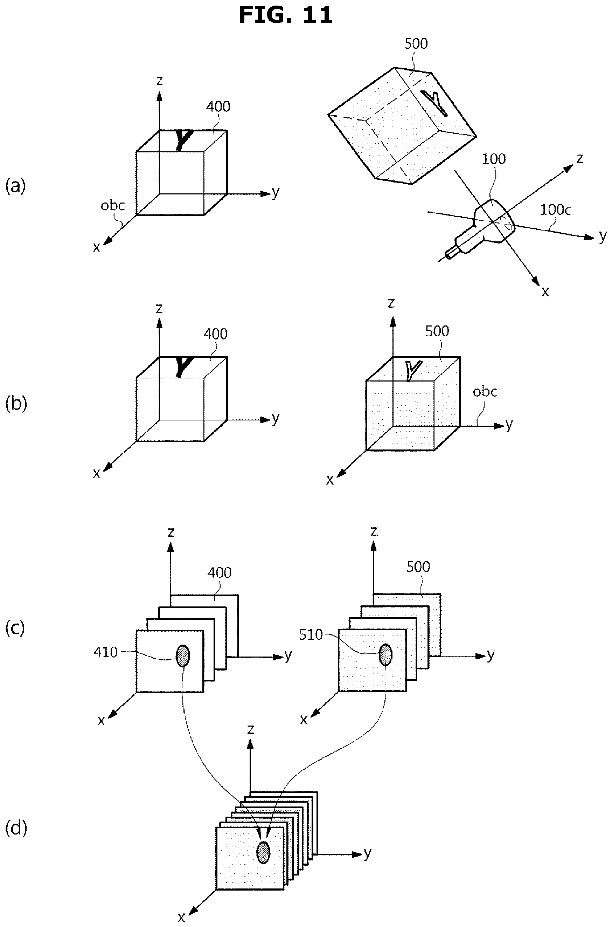

FIG. 11 is a view for describing a method for matching a high-resolution medical image with a reference ultrasound image, according to an embodiment of the present disclosure.

In (a) and (b) of FIG 11, the left images represent high-resolution medical images 400 being 3D volume images, and the right images represent reference ultrasound images 500 being 3D volume images.

Referring to (a) of FIG. 11, the controller 260 may acquire a high-resolution medical image 400 of an object ob including coordinate information stored in the storage 250, and a reference ultrasound image 500 created by the image creator 240.

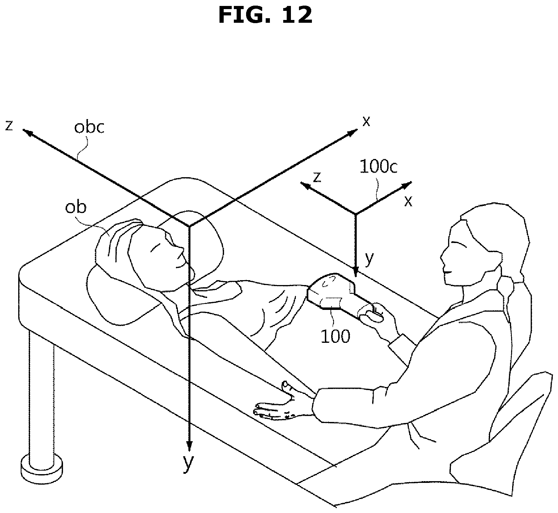

Referring to (b) of FIG. 11, the controller 260 may estimate coordinate information of the reference ultrasound image 500 in the DICOM coordinate system obc, according to coordinate information of the ultrasound probe 100 input by a user. Hereinafter, a method of estimating the coordinate information of the reference ultrasound image 500 in the DICOM coordinate system obc will be described in detail with reference to FIG. 12.

FIG. 12 is a view for describing a method of estimating the coordinate information of the reference ultrasound image 500 in the DICOM coordinate system obc, according to an embodiment of the present disclosure. In FIG. 12, the DICOM coordinate system is used as a reference coordinate system for matching, however, a reference coordinate system for matching is not limited to the DICOM coordinate system.

For example, the directions of the ultrasound probe 100 may be defined in a predetermined coordinate system 100c, and the directions of an object ob may be defined in a predetermined coordinate system obc (for example, the DICOM coordinate system), as shown in FIG. 12.

First, a user may locate the ultrasound probe 100 such that the DICOM coordinate system obc of the object ob is parallel to the coordinate system 100c of the ultrasound probe 100c.

Then, the user may input information about current coordinates of the ultrasound probe 100 through the input 230.

For example, the user may input a command for acquiring coordinate information of the ultrasound probe 100 through the input 230.

Then, the controller 260 may control the position detector 120 to acquire coordinate information of the ultrasound probe 100 at the time when the command for acquiring coordinate information of the ultrasound probe 100 is input.

Successively, the controller 260 may estimate coordinate information of a reference ultrasound image in the DICOM coordinate system obc from the coordinate information of the ultrasound probe 100.

For example, if the user inputs information representing that a positional relationship between the ultrasound probe 100 and the object ob is "parallel" through the input 230, the controller 260 may determine that the coordinate system 100c of the ultrasound probe 100 is identical to the DICOM coordinate system obc, based on the coordinate information of the ultrasound probe 100.

Accordingly, the controller 260 may determine where the reference ultrasound image 500 is located in the DICOM coordinate system obc and which direction the reference ultrasound image 500 has been obtained from.

In this case, since a high-resolution medical image 400 stored in the storage 250 includes coordinate information based on the DICOM coordinate system obc, as described above with reference to FIG. 10, the coordinate system of the high-resolution medical image 400 may match the "coordinate system" of the reference ultrasound image 500 created by the image creator 240.

Meanwhile, in the embodiment of FIG. 12, the ultrasound probe 100 is located such that the DICOM coordinate system obc of the object ob is parallel to the coordinate system of the ultrasound probe 100, however, the user may position the ultrasound probe 100 at another angle, and input information about a positional relationship between the ultrasound probe 100 and the object ob through the input 230.

Successively, the controller 260 may match the reference ultrasound image 500 with the high-resolution medical image 400, based on the estimated coordinate information of the reference ultrasound image 500.

The controller 260 may match the reference ultrasound image 500 with the high-resolution medical image 400, based on the user's input, or automatically.

More specifically, referring again to (c) of FIG. 11, the user may select a first feature point 410 of the high-resolution medical image 400 and a second feature point 510 of the reference ultrasound image 500 corresponding to the first feature point 410, through the input 230.

More specifically, the user may select the first feature point 410 from a section image among a plurality of section images included in the volume image of the high-resolution medical image 400, through the input 230.

Also, the user may select the second feature point 510 from a section image among a plurality of section images included in the volume image of the reference ultrasound image 500, through the input 230.

Meanwhile, the user may select the first feature point 410 and the second feature point 510 from the respective volume images (that is, the high-resolution medical image 400 and the reference ultrasound image 500), through the input 230, without dividing each volume image into a plurality of section images.

Also, the second feature point 510 may be a predetermined point in the reference ultrasound image 500. In this case, the user may check the second feature point 510 displayed through the display 220 to select the first feature point 410 corresponding to the second feature point 510 from the high-resolution medical image 400.

Successively, the controller 260 may match the volume image of the high-resolution medical image 400 with the volume image of the reference ultrasound image 500 based on the first feature point 410 and the second feature point 510. For example, the controller 260 may determine the first feature point 410 and the second feature point 510 as the same point in the object ob.

In the embodiment of FIG. 11, the high-resolution medical image 400 and the reference ultrasound image 500 are volume images, however, the high-resolution medical image 400 and the reference ultrasound image 500 may be section images.

Also, the controller 260 may perform matching based on additional feature points input by the user to thereby improve accuracy of matching.

If matching is completed, the user may move the ultrasound probe 100, and the position detector 120 may acquire coordinate information of the ultrasound probe 100 in real time.

The coordinate information acquired by the position detector 120 is transmitted in real time to the controller 260, and the controller 260 traces coordinate information of a high-resolution medical image corresponding to coordinate information of an ultrasound image that is created in real time, based on the coordinate information received from the position detector 120.

For example, if the ultrasound probe 100 moves by x1 in the x-axis direction from the first feature point 410 of (c) of FIG. 11 so that an ultrasound image is created at a location moved by x1, the controller 260 may trace a location moved by x1 in the x-axis direction from the second feature point 510 to detect a section image of a high-resolution medical image corresponding to the location moved by x1.

The detected high-resolution medical image and the ultrasound image corresponding to the high-resolution medical image may be displayed in real time through the display 220.

Successively, the controller 260 extracts a point of interest input by a user or a predetermined point of interest, and guide movements of the ultrasound probe 100 such that the point of interest or a ROI including the point of interest exists within an irradiation range of the ultrasound probe 100.

The point of interest may be coordinate information of a predetermined location of an object ob, stored in advance, or may be coordinate information of a location of an object ob input through the input 230 according to a user's manipulation, as described above with reference to FIG. 8.

A predetermined ROI may be a region that is within a predetermined distance from a point of interest.

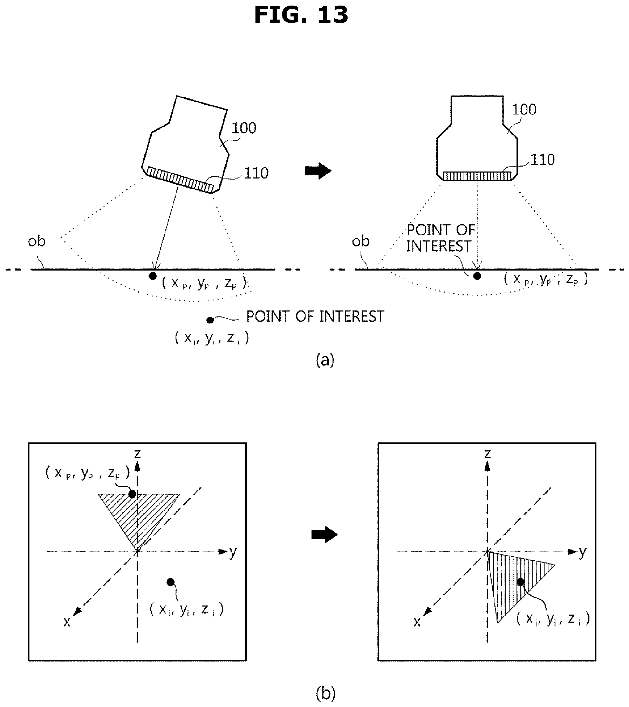

FIG. 13 is a view for describing a method of guiding movements of the ultrasound probe 100, according to an embodiment of the present disclosure.

For example, referring to (a) of FIG. 13, if coordinate information of a point of interest is $(x_i, y_i, z_i)$ in the DICOM coordinate system and coordinate information of a current irradiation focal point of the ultrasound probe 100 is $(x_p, y_p, z_p)$ in the DICOM coordinate system, the controller 260 may control the display 220 to display a marker indicating a current direction of the ultrasound probe 100 for a user, as shown in (b) of FIG. 13, so that coordinate information of an irradiation focal point becomes $(x_i, y_i, z_i)$ in the DICOM coordinate system.

However, the controller 260 may control the display 220 to display, instead of displaying a marker as shown in (b) of FIG. 13, a graph of a +/- bar shape representing an offset from a point of interest, thereby guiding movements of the ultrasound probe 100.

Successively, the controller 260 determines an irradiation focal point of a focus beam, and control the ultrasound probe 100 to irradiate a focus beam at the determined irradiation focal point.

More specifically, the controller 260 may decide an offset between an irradiation focal point and a point of interest in order to determine an irradiation focal point of a focus beam. Hereinafter, an offset between an irradiation focal point and a point of interest based on the DICOM coordinate system will be described.

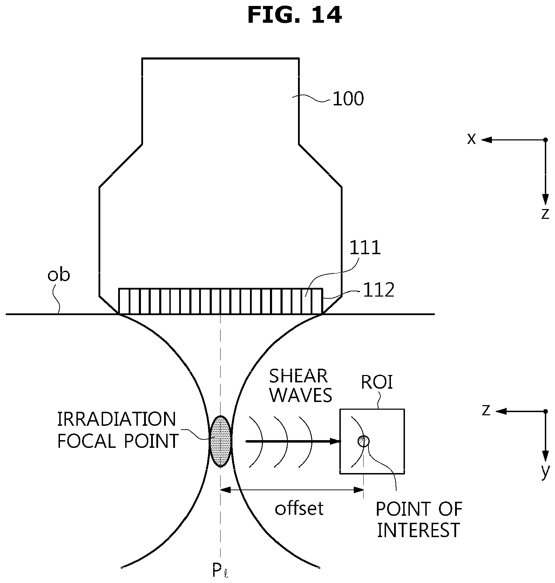

FIG. 14 shows an yz plane of an object for describing an offset between an irradiation focal point and a point of interest, and FIG. 15 shows an xz plane of an object for describing an offset between an irradiation focal point and a point of interest.

If an offset between an irradiation focal point and a point of interest is too short, it is difficult to measure an accurate degree of elasticity of the point of interest since curved shear waves are transferred to the point of interest as seen from the xz plane.

Meanwhile, if an offset between an irradiation focal point and a point of interest is too long, a magnitude of shear waves sufficient to measure a degree of elasticity at the point of interest cannot be ensured due to attenuation of shear waves.

Accordingly, referring to FIGS. 14 and 15, the controller 260 may decide an offset between an irradiation focal point and a point of interest, based on an amount of change in the x-axis direction of shear waves generated from the irradiation focal point.

The controller 260 may decide an offset between an irradiation focal point and a point of interest according to Equation (1) below.

$$\varepsilon = \frac{\partial^2 u_z}{\partial y^2} / \left( \frac{\partial^2 u_z}{\partial x^2} + \frac{\partial^2 u_z}{\partial z^2} \right) < Thres$$

$$(1)$$

where $\varepsilon$ represents an error rate, uz represents displacement of shear waves in the z-axis direction, and *Thres* represents a predetermined threshold value.

As seen from Equation (1), the error rate is proportional to an amount of change in the x-axis direction of the shear waves, and inversely proportional to a sum of an amount of change in the y-axis direction of the shear waves and an amount of change in the z-axis direction of the shear waves.

The controller 260 may decide displacement of shear waves in the z-axis direction such that the error rate is smaller than the predetermined threshold value, and decide a value that is greater than an offset between the displacement and the point of interest, as an offset between the irradiation focal point and the point of interest.

Also, the controller 260 may decide an offset between an irradiation focal point and a point of interest based on a magnitude of shear waves, in order to decide an offset in consideration of attenuation of shear waves.

Also, the controller 260 may decide a depth-direction (that is, y-axis direction) vertical line extending to a point spaced by the decided offset from the point of interest, as a pushing line pi.

Successively, the controller 260 may determine a point which is located at the same depth as the point of interest and which exists on the pushing line pi, as an irradiation focal point.

For example, the controller 260 may determine a point which is located at the same depth as the point of interest in the y-axis direction in the DICOM coordinate system, and which exists on the pushing line pi, as an irradiation focal point.

Also, the controller 260 may determine an irradiation width of the ultrasound probe 100, and set a plurality of irradiation points.

FIG. 16 is a view for describing the number of irradiation focal points of a focus beam according to irradiation widths of ultrasonic waves.

As described above with reference to FIG. 3, the controller 260 may select a range (that is, an activation range) of transducers 111 that are activated, and the activation range may vary according to a predetermined value or a user's input.

The wider activation range, the wider irradiation width ap1 of a focus beam, which is shown in (a) of FIG. 16, and the narrower activation range, the narrower irradiation width ap2 of a focus beam, which is shown in (b) of FIG. 16.

As the irradiation width ap1 of a focus beam is wider, the focusing effect of the focus beam is higher so that the number of irradiation focal points decreases, as shown in (a) of FIG. 16, and as the irradiation width ap2 of a focus beam is narrower, the focusing effect of the focus beam is lower so that the number of irradiation focal points increases, as shown in (b) of FIG. 16,

As the number of irradiation focal points is proportional to the Depth of Field (DOF) of irradiation focal points, if a user inputs a desired DOF value instead of the number of irradiation focal points, the controller 260 may decide an irradiation width ap of a focus beam according to Equation (2), below.

$$DOF = 8 * \left(\frac{F}{ap}\right) * 2\lambda$$

, (2)

where F represents a depth of an irradiation focal point, and $\lambda$ represents a wavelength of a focus beam.

Successively, the controller 260 may determine an irradiation focal point according to a determined location and width of the irradiation focal point, and control the ultrasound probe 100 to irradiate a focus beam to the irradiation focal point.

The controller 260 may control the image creator 240 to create an ultrasound elastic image based on reflected ultrasonic waves, and control the display 220 to display an ultrasound image, a high-resolution medical image, and an ultrasound elastic image.

At this time, the controller 260 may calculate a degree of elasticity of a point of interest using Equation (3) below.

$$\rho \frac{\partial^2 u_z}{\partial t^2} = \mu(x, y, z)\left(\frac{\partial^2 u_z}{\partial x^2} + \frac{\partial^2 u_z}{\partial y^2} + \frac{\partial^2 u_z}{\partial z^2}\right)$$

, (3)

where $\rho$ represents the density of medium, $\mu$ represents a degree of elasticity, and $uz$ represents displacement in the z-axis direction of shear waves.

If the object ob is a human body, $\rho$ may be the density of body tissue.

Also, since an offset between an irradiation focal point and a point of interest is decided such that an amount of change in the x-axis direction of shear waves at the point of interest is sufficiently small (that is, such that shear waves are irradiated in the form of straight lines), as described above with reference to FIG. 15, $\frac{\partial^2 u_z}{\partial y^2}$ may be assumed to be zero.

The controller 260 may measure an amount of change of shear waves passing through the individual points in a ROI over time, and an amount of change of the shear waves with respect to each axis, thereby measuring a degree of elasticity of each point (including a point of interest).

Also, the controller 260 may include, as shown in FIG. 2, a processor 261, ROM 263 which stores control programs for controlling the ultrasound imaging apparatus, and RAM 262 which stores signals or data received from external devices or which is used as a storage space for various tasks that are performed on the ultrasound imaging apparatus.

Also, the controller 260 may include a graphic processing board (not shown) including the processor 261, the RAM 262, or the ROM 263 on a separate circuit board electrically connected to the controller 260.

The processor 261, the RAM 262, and the ROM 263 may be connected to each other through internal buses.

Also, the controller 260 may be a component including the processor 261, the RAM 262, and the ROM 263.

Also, the controller 260 may be a component including the processor 261, the RAM 262, the ROM 263, and a processing board (not shown).

As such, since the ultrasound imaging apparatus displays an ultrasound elastic image for a point of interest together with an ultrasound image and a high-resolution medical image matching with the ultrasound image, it is possible to provide a user with accurate diagnosis about the point of interest.

However, a method of matching an ultrasound image with a high-resolution medical image is not limited to the embodiment as described above, and may be performed in various ways, manually, or automatically.

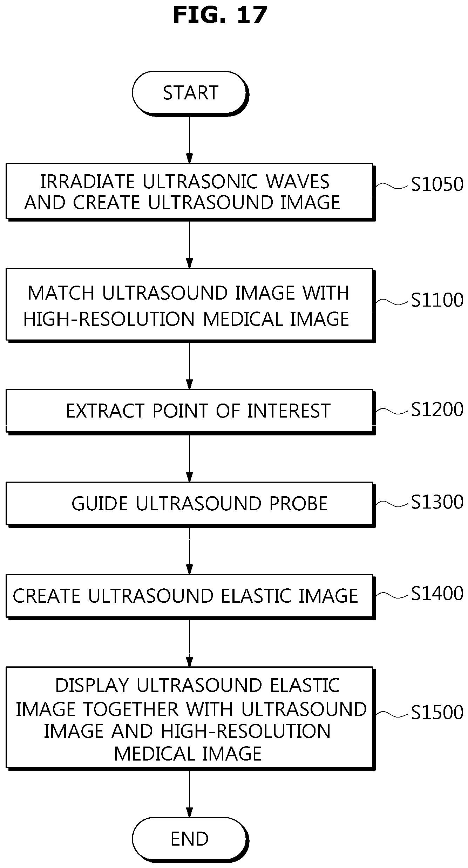

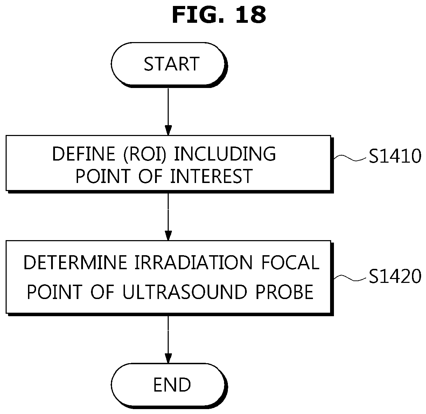

Hereinafter, a method of controlling the ultrasound imaging apparatus will be described with reference to FIGS. 17 and 18.

FIG. 17 is a flowchart illustrating a control method of the ultrasound imaging apparatus, according to an embodiment of the present disclosure, and FIG. 18 is a flowchart illustrating a method of creating an ultrasound elastic image in the ultrasound imaging apparatus, according to an embodiment of the present disclosure.

13

Referring to FIG. 17, the ultrasound imaging apparatus may supply a voltage to the ultrasound probe 100 (see FIG. 2) to irradiate ultrasonic waves to an object ob, and receive ultrasonic waves (hereinafter, referred to as reflected ultrasonic waves) reflected from the object ob to create an ultrasound image, in operation S1050.

Successively, the ultrasound imaging apparatus matches the ultrasound image with a high-resolution medical image, in operation S1100.

As an example of a method of matching an ultrasound image with a high-resolution medical image, there is a method of receiving coordinate information of the ultrasound probe 100 from a user, and receiving a second feature point of an ultrasound image corresponding to a first feature point of a high-resolution medical image to manually match the ultrasound image with the high-resolution medical image.

However, the ultrasound imaging apparatus may match the ultrasound image with the high-resolution medical image automatically, instead of matching the ultrasound image with the high-resolution medical image manually according to a user's input.

Also, the ultrasound image and the high-resolution medical image that match with each other may be displayed through the display 220 for the user.

Successively, the ultrasound imaging apparatus may extract a point of interest set by the user, or a predetermined point of interest, in operation S1200.

The point of interest may be coordinate information of a predetermined point of the object ob, or may be coordinate information of a point of the object ob input through the input 230 according to a user's manipulation.

If a point of interest is input according to a user's manipulation, the user may select a point of a displayed high-resolution medical image or a point of a displayed ultrasound image to thereby input a point of interest.

Successively, the ultrasound imaging apparatus guides movements of the ultrasound probe 100 such that the point of interest is within an ultrasound irradiation range of the ultrasound probe 100, in operation S1300.

For example, the ultrasound imaging apparatus may display a current irradiation range of the ultrasound probe 100 together with the location of the point of interest, for the user. In this case, the user may control the ultrasound probe 100 such that the irradiation range of the ultrasound probe 100 includes the location of the point of interest, while seeing the irradiation range of the ultrasound probe 100.

Also, the ultrasound imaging apparatus may display a graph of a +/- bar shape representing an offset from the point of interest, thereby guiding movements of the ultrasound probe 100.

Successively, the ultrasound imaging apparatus creates an ultrasound elastic image for a ROI, in operation S1400. At this time, the ultrasound imaging apparatus irradiates a focus beam for creating an ultrasound elastic image, to the object ob, after a predetermined time period has elapsed or according to the user's input. An example of a method of creating an ultrasound elastic image will be described in detail with reference to FIG. 18, later.

Successively, the ultrasound imaging apparatus displays the ultrasound elastic image together with the ultrasound image and the high-resolution medical image, for the user, in operation S1500.

For example, the ultrasound elastic image for the point of interest may overlap with the ultrasound image, or may be displayed separately from the ultrasound image and the high-resolution medical image.

Each point of the ROI corresponding to the ultrasound elastic image may have a predetermined color according to a degree of elasticity. For example, a point with a lower degree of elasticity may be displayed with red.

Also, the ultrasound imaging apparatus may display numerals representing degrees of elasticity of the individual points of the ROI.

Hereinafter, a method (operation S1400) in which the ultrasound imaging apparatus creates an ultrasound elastic image will be described as an example with reference to FIG. 18.

First, the ultrasound imaging apparatus may define a ROI including an extracted point of interest, in operation S1410.

The ROI may be a region which is within a predetermined distance from the point of interest. Also, the ROI may be a region set by a user.

Successively, the ultrasound imaging apparatus determines an irradiation focal point of the ultrasound probe 100, in operation S1420.

In order to determine an irradiation focal point of the ultrasound probe 100, the ultrasound imaging apparatus may determine an offset between an irradiation focal point and the point of interest, a depth of the irradiation focal point, and an irradiation width of ultrasonic waves.

For example, the ultrasound imaging apparatus may set a depth of an irradiation focal point to a depth of the point of interest, decide an offset between the irradiation focal point and the point of interest based on an amount of change of shear waves, and decide an irradiation width of ultrasonic waves based on the number of focal points of ultrasonic waves or the DOF of focal points of ultrasonic waves.

According to the embodiments of the present disclosure as described above, a user can see an ultrasound elastic image about abnormal lesions found in a high-resolution medical image or an ultrasound image, together with the high-resolution medical image and the ultrasound image matching with each other.

Also, according to the embodiments of the present disclosure as described above, the user can see a high-resolution medical image with high contrast and high resolution together with an ultrasound elastic image providing quantitative elastic values.

Meanwhile, the control method of the ultrasound imaging apparatus as described above may be embodied as computer-readable code in computer-readable recording medium. The computer-readable recording medium includes any kind of recording device that store data readable by a computer system. For example, the computer-readable recording medium may be ROM, RAM, a magnetic tape, a magnetic disk, flash memory, or an optical data storage device. In addition, the computer-readable recording medium may be distributed to computer systems over a network, in which computer readable codes may be stored and executed in a distributed manner.

It should be understood that the exemplary embodiments described above are merely for illustrative purposes and not for limitation purposes in all aspects. For example, each component described as a single type can be implemented in a distributed type, and components described as distributed can be implemented in a combined form.

**Claims**

1. An ultrasound imaging apparatus comprising:

   an image creator (240) configured to create an ultrasound elastic image that represents a degree of elasticity for a region of interest including a predetermined point of interest of an object;
   a display (220) configured to display the ultrasound elastic image together with an ultrasound image for the object and a high-resolution medical image (400) matching with the ultrasound image;
   an ultrasound probe (100) configured to irradiate a focus beam to an irradiation focal point so as to transfer shear waves from the irradiation focal point to the point of interest;
   a connector (210) connected to an ultrasound probe (100), wherein the connector (210) is configured to receive an ultrasound signal, an ultrasound elastic signal and a location signal of the ultrasound probe (100), from the ultrasound probe (100), and to transfer the ultrasound signal and the ultrasound elastic signal to the image creator (240), the image creator (240) is configured to create the ultrasound image based on the ultrasound signal and the ultrasound elastic image based on the ultrasound elastic signal; and
   a controller (260) configured to match the ultrasound image with a high-resolution medical image and further to detect a high-resolution medical image (400) corresponding to the ultrasound image in real time, based on the location signal of the ultrasound probe (100);
   **characterized in that**
   the controller (260) is configured to determine the irradiation focal point of the ultrasound probe (100) in order to acquire the ultrasound elastic image, and
   to guide an operator how to move the ultrasound probe (100) so that the point of interest exists in an irradiation range of the ultrasound probe (100).

2. The ultrasound imaging apparatus according to claim 1, wherein the controller (260) decides an offset between the irradiation focal point and the point of interest in order to determine the irradiation focal point.

3. The ultrasound imaging apparatus according to claim 2, wherein the controller (260) decides the offset between the irradiation focal point and the point of interest, based on an amount of change in axis direction of shear waves irradiated from the irradiation focal point.

4. The ultrasound imaging apparatus according to claim 2, wherein the controller (260) determines, as the irradiation focal point, a point located at the same depth as the point of interest and spaced by the offset in a lateral direction from the point of interest.

5. The ultrasound imaging apparatus according to claim 1, wherein the controller (260) determines an irradiation width of the ultrasound probe (100) according to a predetermined number of irradiation focal points.

6. The ultrasound imaging apparatus according to claim 1, wherein the high-resolution medical image (400) is at least one image of a magnetic resonance image and an X-ray image.

7. The ultrasound imaging apparatus according to claim 1, further comprising:
   a storage (250) in which at least one high-resolution medical image (400) is stored.

**8.** The ultrasound imaging apparatus according to claim 7, further comprising an input (230) configured to receive a user's input (230) of inputting at least one of coordinate information and a feature point of the ultrasound image and at least one of coordinate information and a feature point of the high-resolution medical image,
wherein the controller (260) matches the ultrasound image with the high-resolution medical image, based on the user's input (230).

**9.** The ultrasound imaging apparatus according to claim 1, wherein the display (200) displays a predetermined color according to a degree of elasticity of the predetermined point of interest, as the ultrasound elastic image.

**10.** The ultrasound imaging apparatus according to claim 1, wherein the display (200) displays a spectral image based on a degree of elasticity of the predetermined point of interest, as the ultrasound elastic image.

**11.** The ultrasound imaging apparatus according to claim 1, further comprising an input (230) configured to receive a user's input (230) of setting a point in the ultrasound image or the high-resolution medical image (400) to a point of interest,
wherein the image creator (240) creates an ultrasound elastic image for the point of interest set according to the user's input (230).

**12.** A control method of an ultrasound imaging apparatus according to claim 1, comprising:

creating an ultrasound image for an object;
displaying the ultrasound image together with a high-resolution medical image (400) stored in advance;
creating an ultrasound elastic image that represents a degree of elasticity for a region of interest including a predetermined point of interest of the object; and
displaying the ultrasound elastic image together with the ultrasound image and a high-resolution medical image (400) matching with the ultrasound image and detecting a high-resolution medical image corresponding to the ultrasound image in real time, based on the location signal of the ultrasound probe,
wherein the creating of the ultrasound elastic image comprises
determining an irradiation focal point of the ultrasound probe (100) connected to the ultrasound imaging apparatus; and
irradiating via the ultrasound probe a focus beam to the irradiation focal point so as to transfer shear waves from the irradiation focal point to the point of interest; guiding an operator how to move the ultrasound probe (100) so that the point of interest exists in an irradiation range of the ultrasound probe (100).

**13.** The control method according to claim 12, wherein the determining of the irradiation focal point of the ultrasound probe (100) comprises deciding an offset between the irradiation focal point and the point of interest.

**14.** The control method according to claim 12, wherein the determining of the irradiation focal point of the ultrasound probe (100) comprises determining an irradiation width of the ultrasound probe (100) according to a predetermined number of irradiation focal points.

**15.** The control method according to claim 12, before displaying the ultrasound elastic image, further comprising matching the ultrasound image with the high-resolution medical image.

**16.** The ultrasound imaging apparatus according to claim 1,
wherein the controller is configured to control the display to display a marker indicating a current direction of the ultrasound probe for a user, so that the coordinate information of the irradiation focal point $(x_p,y_p,z_p)$ in the DICOM coordinate system becomes the coordinate information of the point of interest $(x_i,y_i,z_i)$ in the DICOM coordinate system.

**Patentansprüche**

**1.** Ultraschallabbildungsvorrichtung, welche Folgendes aufweist:

einen Bilderzeuger (240), der dafür vorgesehen ist, ein elastisches Ultraschallbild zu erzeugen, das einen Grad der Elastizität für einen interessierenden Bereich, der einen bestimmten interessierenden Punkt eines Objekts beinhaltet, repräsentiert;

eine Anzeige (220), die dafür vorgesehen ist, das elastische Ultraschallbild zusammen mit einem Ultraschallbild für das Objekt und einem hochauflösenden medizinischen Bild (400), das mit dem Ultraschallbild zusammenpasst, anzuzeigen;

eine Ultraschallsonde (100), die dafür vorgesehen ist, einen Fokusstrahl an einen Bestrahlungs-Fokalpunkt auszustrahlen, um Transversalwellen von dem Bestrahlungs-Fokalpunkt zu dem interessierenden Punkt zu transferieren;

einen Verbinder (210), der mit einer Ultraschallsonde (100) verbunden ist, wobei der Verbinder (210) dafür vorgesehen ist, ein Ultraschallsignal, ein elastisches Ultraschallsignal und ein Positionssignal der Ultraschallsonde (100) von der Ultraschallsonde (100) zu empfangen und das Ultraschallsignal und das elastische Ultraschallsignal zu dem Bilderzeuger (240) zu übertragen, wobei der Bilderzeuger (240) dafür vorgesehen ist, das Ultraschallbild, basierend auf dem Ultraschallsignal und dem elastischen Ultraschallbild, das auf dem elastischen Ultraschallsignal basiert, zu erzeugen; und

ein Steuergerät (260), das dafür vorgesehen ist, das Ultraschallbild mit einem hochauflösenden medizinischen Bild abzustimmen und des Weiteren ein hochauflösendes medizinisches Bild (400), das mit dem Ultraschallbild korrespondiert, in Echtzeit zu detektieren, und zwar basierend auf dem Positionssignal der Ultraschallsonde (100);

**dadurch gekennzeichnet, dass**

das Steuergerät (260) dafür vorgesehen ist, den Bestrahlungs-Fokalpunkt der Ultraschallsonde (100) zu ermitteln, um das elastische Ultraschallbild zu erlangen; und

eine Bedienperson anzuleiten, wie die Ultraschallsonde (100) bewegt werden soll, so dass der interessierende Punkt in einem Bestrahlungsbereich der Ultraschallsonde (100) existiert.

2. Ultraschallabbildungsvorrichtung nach Anspruch 1, wobei das Steuergerät (260) einen Versatz zwischen dem Bestrahlungs-Fokalpunkt und dem interessierenden Punkt bestimmt, um den Bestrahlungs-Fokalpunkt zu ermitteln.

3. Ultraschallabbildungsvorrichtung nach Anspruch 2, wobei das Steuergerät (260) den Versatz zwischen dem Bestrahlungs-Fokalpunkt und dem interessierenden Punkt basierend auf einer Menge an Veränderungen in Achsrichtung von Transversalwellen, die von dem Bestrahlungs-Fokalpunkt ausgestrahlt wurden, bestimmt.

4. Ultraschallabbildungsvorrichtung nach Anspruch 2, wobei das Steuergerät (260) als den Bestrahlungs-Fokalpunkt einen Punkt ermittelt, der auf derselben Tiefe wie der interessierende Punkt liegt und um den Versatz in einer seitliche Richtung von dem interessierenden Punkt beabstandet ist.

5. Ultraschallabbildungsvorrichtung nach Anspruch 1, wobei das Steuergerät (260) eine Bestrahlungsbreite der Ultraschallsonde (100) gemäß einer bestimmten Anzahl an Bestrahlungs-Fokalpunkten ermittelt.

6. Ultraschallabbildungsvorrichtung nach Anspruch 1, wobei das hochauflösende medizinische Bild (400) wenigstens eines von einem magnetischen Resonanzbild oder einem Röntgenbild ist.

7. Ultraschallabbildungsvorrichtung nach Anspruch 1, welche des Weiteren Folgendes aufweist:
einen Speicher (250), in dem wenigstens ein hochauflösendes medizinisches Bild (400) gespeichert ist.

8. Ultraschallabbildungsvorrichtung nach Anspruch 7, welche des Weiteren eine Eingabe (230) aufweist, die dafür vorgesehen ist, eine Eingabe (230) eines Benutzers zu empfangen, zum Eingeben wenigstens eines von Koordinateninformationen und einem Merkmalspunkt des Ultraschallbilds und wenigstens eines von Koordinateninformationen und einem Merkmalsbild des hochauflösenden medizinischen Bilds,
wobei das Steuergerät (260) das Ultraschallbild mit dem hochauflösenden medizinischen Bild basierend auf der Eingabe (230) des Benutzers abstimmt.

9. Ultraschallabbildungsvorrichtung nach Anspruch 1, wobei die Anzeige (200) eine bestimmte Farbe gemäß einem Grad der Elastizität des bestimmten interessierenden Punkts als das elastische Ultraschallbild anzeigt.

10. Ultraschallabbildungsvorrichtung nach Anspruch 1, wobei die Anzeige (200) ein Spektralbild basierend auf einem Grad der Elastizität des bestimmten interessierenden Punkts als das elastische Ultraschallbild anzeigt.

11. Ultraschallabbildungsvorrichtung nach Anspruch 1, welche des Weiteren eine Eingabe (230) aufweist, die dafür vorgesehen ist, eine Eingabe (230) eines Benutzers zur Festlegung eines Punkts in dem Ultraschallbild oder dem hochauflösenden medizinischen Bild (400) als einen interessierenden Punkt zu empfangen,

wobei der Bilderzeuger (240) ein elastisches Ultraschallbild für den gemäß der Eingabe (230) des Benutzers festgelegten interessierenden Punkt erzeugt.

12. Verfahren zur Steuerung einer Ultraschallabbildungsvorrichtung nach Anspruch 1, welches Folgendes aufweist:

Erzeugen eines Ultraschallbilds für ein Objekt;
Anzeigen des Ultraschallbilds zusammen mit einem hochauflösenden medizinischen Bild (400), das im Voraus gespeichert wurde;
Erzeugen eines elastischen Ultraschallbilds, das einen Grad der Elastizität für einen interessierenden Bereich, der einen bestimmten interessierenden Punkt des Objekts beinhaltet, repräsentiert; und
Anzeigen des elastischen Ultraschallbilds zusammen mit dem Ultraschallbild und einem hochauflösenden medizinischen Bild (400), das mit dem Ultraschallbild zusammenpasst, und Detektieren eines hochauflösenden medizinischen Bilds, das mit dem Ultraschallbild korrespondiert, in Echtzeit basierend auf dem Positionssignal der Ultraschallsonde,
wobei das Erzeugen des elastischen Ultraschallbilds Folgendes aufweist:

Ermitteln eines Bestrahlungs-Fokalpunkts der Ultraschallsonde (100), die mit der Ultraschallabbildungsvorrichtung verbunden ist; und
Ausstrahlen eines Fokusstrahls auf den Bestrahlungs-Fokalpunkt mittels der Ultraschallsonde, um Transversalwellen von dem Bestrahlungs-Fokalpunkt zu dem interessierenden Punkt zu übertragen;
Anleiten (?) einer Bedienperson, wie die Ultraschallsonde (100) bewegt werden soll, so dass der interessierende Punkt in einem Bestrahlungsbereich der Ultraschallsonde (100) existiert.

13. Verfahren zur Steuerung nach Anspruch 12, wobei das Ermitteln des Bestrahlungs-Fokalpunkts der Ultraschallsonde (100) das Bestimmen (?) eines Versatzes zwischen dem Bestrahlungs-Fokalpunkt und dem interessierenden Punkt beinhaltet.

14. Verfahren zur Steuerung nach Anspruch 12, wobei das Ermitteln des Bestrahlungs-Fokalpunkts der Ultraschallsonde (100) das Ermitteln einer Bestrahlungsbreite der Ultraschallsonde (100) gemäß einer bestimmten Anzahl von Bestrahlungs-Fokalpunkten beinhaltet.

15. Verfahren zur Steuerung nach Anspruch 12, welches vor dem Anzeigen des elastischen Ultraschallbilds des Weiteren das Abstimmen des Ultraschallbilds mit dem hochauflösenden medizinischen Bild beinhaltet.

16. Ultraschallabbildungsvorrichtung nach Anspruch 1,
wobei das Steuergerät dafür vorgesehen ist, die Anzeige so zu steuern, dass sie eine Markierung anzeigt, die eine Stromrichtung der Ultraschallsonde für einen Benutzer anzeigt, so dass die Koordinateninformationen des Bestrahlungs-Fokuspunkts ($x_p$, $y_p$, $z_p$) in dem DICOM-Koordinatensystem die Koordinateninformationen des interessierenden Punkts ($x_i$, $y_i$, $z_i$) in dem DICOM-Koordinatensystem werden.

## Revendications

1. Appareil d'imagerie par ultrasons comprenant :

un créateur d'image (240) configuré pour créer une image élastique ultrasonore qui représente un degré d'élasticité pour une région d'intérêt incluant un point d'intérêt prédéterminé d'un objet ;
un affichage (220) configuré pour afficher l'image élastique ultrasonore avec une image ultrasonore pour l'objet et une image médicale haute résolution (400) coïncidant avec l'image ultrasonore ;
une sonde à ultrasons (100) configurée pour irradier un faisceau de focalisation vers un point focal d'irradiation de manière à transférer des ondes de cisaillement du point focal d'irradiation au point d'intérêt ;
un connecteur (210) connecté à une sonde à ultrasons (100), dans lequel le connecteur (210) est configuré pour recevoir un signal ultrasonore, un signal élastique ultrasonore et un signal de localisation de la sonde à ultrasons (100), provenant de la sonde à ultrasons (100), et pour transférer le signal ultrasonore et le signal élastique ultrasonore au créateur d'image (240), le créateur d'image (240) est configuré pour créer l'image ultrasonore sur la base du signal ultrasonore et l'image élastique ultrasonore sur la base du signal élastique ultrasonore ; et
un dispositif de commande (260) configuré pour faire coïncider l'image ultrasonore avec une image médicale haute résolution et pour détecter en outre une image médicale haute résolution (400) correspondant à l'image ultrasonore en temps réel, sur la base du signal de localisation de la sonde à ultrasons (100) ;

**caractérisé en ce que**

le dispositif de commande (260) est configuré pour déterminer le point focal d'irradiation du sonde à ultrasons (100) afin d'acquérir l'image élastique ultrasonore, et

guider un opérateur sur la façon de déplacer la sonde à ultrasons (100) de sorte que le point d'intérêt existe dans une plage d'irradiation de la sonde à ultrasons (100).

2. Appareil d'imagerie par ultrasons selon la revendication 1, dans lequel le dispositif de commande (260) décide d'un décalage entre le point focal d'irradiation et le point d'intérêt afin de déterminer le point focal d'irradiation.

3. Appareil d'imagerie par ultrasons selon la revendication 2, dans lequel le dispositif de commande (260) décide du décalage entre le point focal d'irradiation et le point d'intérêt, sur la base d'une quantité de changement de direction axiale d'ondes de cisaillement irradiées à partir du point focal d'irradiation.

4. Appareil d'imagerie par ultrasons selon la revendication 2, dans lequel le dispositif de commande (260) détermine, en tant que point focal d'irradiation, un point situé à la même profondeur que le point d'intérêt et espacé par le décalage dans une direction latérale par rapport au point d'intérêt.

5. Appareil d'imagerie par ultrasons selon la revendication 1, dans lequel le dispositif de commande (260) détermine une largeur d'irradiation de la sonde à ultrasons (100) selon un nombre prédéterminé de points focaux d'irradiation.

6. Appareil d'imagerie par ultrasons selon la revendication 1, dans lequel l'image médicale haute résolution (400) est au moins une image d'une image par résonance magnétique et d'une image à rayons X.

7. Appareil d'imagerie par ultrasons selon la revendication 1, comprenant en outre :
un stockage (250) dans lequel au moins une image médicale haute résolution (400) est stockée.

8. Appareil d'imagerie par ultrasons selon la revendication 7, comprenant en outre une entrée (230) configurée pour recevoir une entrée utilisateur (230) consistant à entrer au moins l'un parmi des informations de coordonnées et un point caractéristique de l'image ultrasonore et au moins l'un parmi des informations de coordonnées et un point caractéristique de l'image médicale haute résolution,
dans lequel le dispositif de commande (260) fait coïncider l'image ultrasonore avec l'image médicale haute résolution, sur la base de l'entrée utilisateur (230).

9. Appareil d'imagerie par ultrasons selon la revendication 1, dans lequel l'affichage (200) affiche une couleur prédéterminée selon un degré d'élasticité du point d'intérêt prédéterminé, en tant qu'image élastique ultrasonore.

10. Appareil d'imagerie par ultrasons selon la revendication 1, dans lequel l'affichage (200) affiche une image spectrale basée sur un degré d'élasticité du point d'intérêt prédéterminé, en tant qu'image élastique ultrasonore.

11. Appareil d'imagerie par ultrasons selon la revendication 1, comprenant en outre une entrée (230) configurée pour recevoir une entrée utilisateur (230) consistant à établir un point dans l'image ultrasonore ou l'image médicale haute résolution (400) sur un point d'intérêt,
dans lequel le créateur d'image (240) crée une image élastique ultrasonore pour le point d'intérêt établi en fonction de l'entrée utilisateur (230).

12. Procédé de commande d'un appareil d'imagerie par ultrasons selon la revendication 1, comprenant les étapes consistant à :

créer une image ultrasonore pour un objet ;
afficher l'image ultrasonore ensemble avec une image médicale haute résolution (400) stockée à l'avance ;
créer une image élastique ultrasonore qui représente un degré d'élasticité pour une région d'intérêt incluant un point d'intérêt prédéterminé de l'objet ; et
afficher l'image élastique ultrasonore ensemble avec l'image ultrasonore et une image médicale haute résolution (400) coïncidant avec l'image ultrasonore et détecter une image médicale haute résolution correspondant à l'image ultrasonore en temps réel, sur la base du signal de localisation de la sonde à ultrasons,
dans lequel la création de l'image élastique ultrasonore comprend

la détermination d'un point focal d'irradiation de la sonde à ultrasons (100) reliée à l'appareil d'imagerie par ultrasons ; et

l'irradiation via la sonde à ultrasons d'un faisceau de focalisation vers le point focal d'irradiation de manière à transférer des ondes de cisaillement du point focal d'irradiation au point d'intérêt ;

le guidage d'un opérateur sur la façon de déplacer la sonde à ultrasons (100) de sorte que le point d'intérêt existe dans une plage d'irradiation de la sonde à ultrasons (100).

13. Procédé de commande selon la revendication 12, dans lequel la détermination du point focal d'irradiation de la sonde à ultrasons (100) comprend la décision d'un décalage entre le point focal d'irradiation et le point d'intérêt.

14. Procédé de commande selon la revendication 12, dans lequel la détermination du point focal d'irradiation de la sonde à ultrasons (100) comprend la détermination d'une largeur d'irradiation de la sonde à ultrasons (100) en fonction d'un nombre prédéterminé de points focaux d'irradiation.

15. Procédé témoin selon la revendication 12, avant l'affichage de l'image élastique ultrasonore, comprenant en outre la mise en coïncidence de l'image ultrasonore avec l'image médicale haute résolution.

16. Appareil d'imagerie par ultrasons selon la revendication 1,
dans lequel le dispositif de commande est configuré pour commander l'affichage afin d'afficher un marqueur indiquant une direction actuelle de la sonde à ultrasons pour un utilisateur, de sorte que les informations de coordonnées du point focal d'irradiation $(x_p, y_p, z_p)$ dans le système de coordonnées DICOM deviennent les informations de coordonnées du point d'intérêt $(x_i, y_i, z_i)$ dans le système de coordonnées DICOM.

# FIG. 1

EP 3 017 766 B1

**FIG. 2**

100

120

POSITION
DETECTOR

OBJECT — ob

110

TRANSDUCER
MODULE

200

210 — CONNECTOR

260

240 — IMAGE
CREATOR

CONTROLLER

270

POWER SOURCE

250 — STORAGE

PROCESSOR — 261

230

220 — DISPLAY

RAM    ROM

INPUT

262    263

# FIG. 3

(a)

(b)

# FIG. 4

# FIG. 5

FIG. 6

EP 3 017 766 B1

(a)            (b)            (c)

# FIG. 7

(a)

(b)

EP 3 017 766 B1

# FIG. 8

220

ROI

ULTRASOUND
ELASTIC
IMAGE

ULTRASOUND
IMAGE

HIGH-RESOLUTION
MEDICAL IMAGE

DEGREE OF
ELASTICITY
OF ROI

EP 3 017 766 B1

# FIG. 9

220

ROI

ULTRASOUND
ELASTIC
IMAGE

POINT OF
INTEREST

ULTRASOUND
IMAGE

HIGH-RESOLUTION
MEDICAL IMAGE

DEGREE OF
ELASTICITY
OF ROI

EP 3 017 766 B1

# FIG. 10

HIGH-RESOLUTION MEDICAL IMAGE

# FIG. 11

# FIG. 12

# FIG. 13

(a)

(b)

# FIG. 14

# FIG. 15

IRRADIATION FOCAL POINT

POINT OF INTEREST

offset

X

Z

# FIG. 16

~100

ap1

IRRADIATION FOCAL POINT

SHEAR WAVES

DOF

(a)

~100

ap2

SHEAR WAVES

DOF

(b)

# FIG. 17

```
                    START

         IRRADIATE ULTRASONIC WAVES          S1050
         AND CREATE ULTRASOUND IMAGE

         MATCH ULTRASOUND IMAGE WITH         S1100
         HIGH-RESOLUTION MEDICAL IMAGE

         EXTRACT POINT OF INTEREST           S1200

         GUIDE ULTRASOUND PROBE              S1300

         CREATE ULTRASOUND ELASTIC IMAGE     S1400

         DISPLAY ULTRASOUND ELASTIC
         IMAGE TOGETHER WITH ULTRASOUND      S1500
         IMAGE AND HIGH-RESOLUTION
         MEDICAL IMAGE

                    END
```

# FIG. 18

START

DEFINE (ROI) INCLUDING
POINT OF INTEREST — S1410

DETERMINE IRRADIATION FOCAL
POINT OF ULTRASOUND PROBE — S1420

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012133663 A1
- US 2013116542 A1
- WO 2014046263 A1
- JP 2013135738 A
- US 2012172710 A1
- CN 104055536 A
- WO 2013153968 A1
- US 2011066030 A1
- WO 2014162966 A1